# FASCICULE DE BREVET EUROPEEN

(11) **EP 3 230 182 B1**
(45) Date de publication et mention de la délivrance du brevet: **30.01.2019**
(21) Numéro de dépôt: 15817119.9
(22) Date de dépôt: 11.12.2015
(51) Int. Cl.: B65G 43/08

(54) **PROCÉDÉ ET SYSTÈME DE LOCALISATION D'UN OBJET INSTRUMENTÉ TRANSPORTÉ LE LONG D'UN PARCOURS PAR UNE MACHINE DE CONVOI**
VERFAHREN UND SYSTEM ZUR ORTUNG EINES INSTRUMENTIERTEN OBJEKTS, DAS VON EINER FÖRDERMASCHINE ENTLANG EINES PFADS TRANSPORTIERT WIRD
METHOD AND SYSTEM FOR LOCATING AN INSTRUMENTED OBJECT TRANSPORTED ALONG A PATH BY A CONVEYOR MACHINE

(30) Priorité: 12.12.2014 FR 1462311
(43) Date de publication de la demande: 18.10.2017
(73) Titulaire: Commissariat à l'Énergie Atomique et aux Énergies Alternatives, 75015 Paris (FR)
(72) Inventeur: CARMONA, Mikaël, 38570 Tencin (FR); BILLERES, Malvina, 38000 Grenoble (FR); PERIS Y SABORIT, Laure, 38000 Grenoble (FR)
(74) Mandataire: Novaimo
(86) Numéro de dépôt international: PCT/EP2015/079381
(87) Numéro de publication internationale: WO 2016/092064

(56) Documents cités:
- US-A1- 2007 114 110
- US-A1- 2010 010 664
- US-A1- 2010 245 105
- US-B1- 8 260 574

## Description

L'invention concerne un procédé et un système de localisation d'un objet instrumenté transporté le long d'un parcours par une machine de convoi M, l'objet étant doté d'un instrument de mesure d'au moins une grandeur physique donnée.

Une machine de convoi M d'objets, par exemple un colis, une lettre, un déchet ou un produit en cours de fabrication, comprend généralement plusieurs modules successifs destinés à remplir différentes fonctions et/ou à exécuter différentes actions relativement à l'objet convoyé.

La machine de convoi M peut avoir une anomalie et il convient de localiser celle-ci. Il peut également être souhaitable de contrôler le parcours de convoi d'un objet afin de vérifier certains paramètres de réglage de la machine. Or, durant son transport par la machine de convoi M, dans un environnement au moins partiellement confiné, il est difficile d'avoir accès à l'objet. Il en résulte qu'il est également difficile de localiser l'objet durant son transport par la machine de convoi M. US2010/0245105 divulgue un procédé de localisation d'un objet instrumenté transporté le long d'un parcours par une machine de convoi.

La présente invention vient améliorer la situation.

A cet effet, l'invention concerne un procédé de localisation d'un objet instrumenté transporté le long d'un parcours par une machine de convoi, ledit objet étant doté d'un système de mesure d'au moins une grandeur physique donnée, comprenant une étape de mesure, par le système de mesure, de l'évolution temporelle de ladite grandeur physique durant le transport de l'objet le long du parcours de convoi, une étape de reconnaissance d'une pluralité d'empreintes, chaque empreinte correspondant à une caractéristique de parcours dans l'évolution temporelle de la grandeur physique mesurée, une étape de détermination d'une chronologie de caractéristiques de parcours correspondant à une succession d'empreintes reconnues et une étape de localisation de l'objet lors de laquelle on détermine une correspondance entre la chronologie de caractéristiques de parcours déterminée et une représentation spatiale prédéterminée relative à la machine de convoi.

Selon l'invention, les mesures de grandeur(s) physique(s) réalisées par l'objet instrumenté permettent d'identifier des caractéristiques particulières du parcours de l'objet. En effet, certaines caractéristiques particulières de parcours laissent des empreintes, ou signatures, distinguables dans l'évolution temporelle d'une ou plusieurs grandeurs physiques mesurées. L'objet peut être localisé à partir d'une ou plusieurs caractéristiques de parcours identifiées à l'aide d'une ou plusieurs empreintes reconnues. La localisation peut consister à déterminer où se trouve l'objet le long d'un parcours de convoi à un instant donné ou, dans le cas d'une machine de convoi ayant plusieurs parcours possibles, à déterminer le parcours emprunté par l'objet.

Avantageusement, on mesure l'évolution temporelle de l'une au moins des grandeurs physiques du groupe comportant une accélération, une distance, une inclinaison, un champ magnétique, une position angulaire, une température, un son et une pression.

Avantageusement encore, la succession d'empreintes reconnues comprend différents types d'empreintes, notamment des empreintes du groupe comportant une accélération, une distance, une inclinaison, un champ magnétique, une position angulaire, une température, un son et une pression.

Dans un mode de réalisation particulier, lors de l'étape de reconnaissance, on reconnaît l'une au moins des empreintes du groupe comportant un pic d'intensité de champ magnétique mesuré correspondant à un moteur électrique, un pic de distance mesurée correspondant à une transition d'un module à un autre module de la machine de convoi et un plateau d'une accélération mesurée correspondant à un virage.

On peut mesurer les évolutions temporelles d'une pluralité de grandeurs physiques.

Avantageusement, l'étape de reconnaissance est réalisée à l'aide d'une table de données contenant, pour chaque caractéristique d'un ensemble de caractéristiques prédéfinies de parcours, des données de caractérisation de l'empreinte correspondant à ladite caractéristique de parcours.

Dans un mode de réalisation particulier, la machine de convoi comportant une pluralité de parcours de convoi possibles, lors de l'étape de localisation, on détermine le parcours emprunté par l'objet instrumenté, parmi la pluralité de parcours de convoi possibles, à partir de la chronologie de caractéristiques de parcours déterminée.

Le procédé peut comprendre une étape de contrôle du bon fonctionnement de la machine de convoi à partir de la chronologie de caractéristiques de parcours déterminée. Une application envisageable du procédé de localisation de l'invention est de contrôler le fonctionnement de la machine de convoi.

Le procédé peut également comprendre une étape de détection d'une anomalie dans le parcours de convoi de l'objet, basée sur une analyse de l'évolution temporelle d'une grandeur physique mesurée particulière.

Avantageusement, il comprend une étape de transmission de données d'évolution de la grandeur physique mesurée vers un dispositif d'analyse extérieur et les étapes de reconnaissance d'au moins une empreinte et de localisation de l'objet sont exécutées par le dispositif d'analyse extérieur.

L'invention concerne aussi un système de localisation d'un objet transporté le long d'un parcours par une machine de convoi, caractérisé en ce qu'il comprend un objet instrumenté doté d'un système de mesure d'au moins une grandeur physique donnée, agencé pour mesurer l'évolution temporelle de ladite grandeur physique durant le transport de l'objet le long du parcours de convoi, et un dispositif d'analyse extérieur comportant un module de reconnaissance adapté pour reconnaître une pluralité d'empreintes, chaque empreinte correspondant à une caractéristique de parcours dans l'évolution temporelle de la grandeur physique mesurée, un module de détermination d'une chronologie de caractéristiques de parcours correspondant à une succession d'empreintes reconnues et un module de localisation de l'objet adapté pour établir une correspondance entre la chronologie de caractéristiques de parcours déterminée et une représentation spatiale prédéterminée relative à la machine de convoi stockée en mémoire.

Le système comprend avantageusement tout ou partie des caractéristiques suivantes :
- le dispositif d'analyse extérieur comprend une mémoire de stockage d'une table de données contenant, pour chaque caractéristique d'un ensemble de caractéristiques prédéfinies de parcours, des données de caractérisation d'une empreinte correspondant à ladite caractéristique de parcours ;
- le dispositif d'analyse extérieur comprend un module de contrôle du bon fonctionnement de la machine de convoi à partir de la chronologie de caractéristiques de parcours déterminée ;
- il comprend un module de détection d'une anomalie, agencé pour détecter une anomalie dans le parcours de convoi de l'objet par analyse de l'évolution temporelle d'une grandeur physique mesurée particulière.

L'invention concerne aussi un dispositif d'analyse destiné à localiser un objet instrumenté transporté le long d'un parcours par une machine de convoi, ledit objet instrumenté étant doté d'un système de mesure d'au moins une grandeur physique donnée, agencé pour mesurer l'évolution temporelle de ladite grandeur physique durant le transport de l'objet le long du parcours de convoi, ledit dispositif d'analyse comprenant une interface de communication avec le système de mesure destinée à recevoir des données mesurées relatives à l'évolution temporelle de ladite grandeur physique durant le transport de l'objet le long du parcours de convoi, un module de reconnaissance adapté pour reconnaître une pluralité d'empreintes, chaque empreinte correspondant à une caractéristique de parcours dans l'évolution temporelle de la grandeur physique mesurée, un module de détermination d'une chronologie de caractéristiques de parcours correspondant à une succession d'empreintes reconnues et un module de localisation de l'objet adapté pour établir une correspondance entre la chronologie de caractéristiques de parcours déterminée et une représentation spatiale prédéterminée relative à la machine de convoi stockée en mémoire.

L'invention sera mieux comprise à l'aide de la description suivante d'un mode de réalisation particulier du procédé de localisation d'un objet instrumenté transporté par une machine de convoi M, en référence aux dessins annexés sur lesquels :
- La figure 1 représente, à titre d'exemple illustratif, une portion d'un parcours de convoi le long duquel un objet instrumenté est destiné à être transporté ;
- La figure 2 représente un schéma bloc fonctionnel d'un instrument de mesure porté par un objet ;
- La figure 3 représente un organigramme des étapes du procédé de localisation selon un mode de réalisation particulier de l'invention ;
- La figure 4 représente un schéma bloc fonctionnel d'un dispositif d'analyse extérieur ;
- La figure 5 représente l'évolution temporelle d'une distance mesurée durant le transport d'un objet instrumenté par la machine de convoi M ;
- La figure 6A représente l'évolution temporelle d'une accélération mesurée durant le transport d'un objet instrumenté par la machine de convoi M le long d'un parcours représenté partiellement sur la figure 6C ;
- La figure 6B représente un zoom d'une partie de l'évolution temporelle d'accélération de la figure 6A ;
- La figure 7 représente l'évolution temporelle d'un champ magnétique mesuré durant le transport d'un objet instrumenté par la machine de convoi M ;

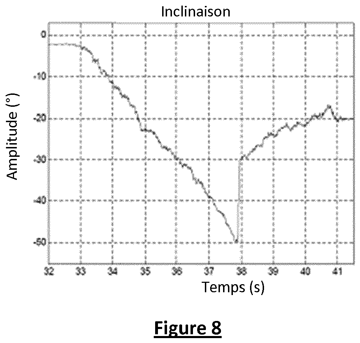

- La figure 8 représente l'évolution temporelle d'une inclinaison mesurée durant le transport d'un objet instrumenté par la machine de convoi M.

L'invention concerne un procédé de localisation d'un objet instrumenté transporté le long d'un parcours par une machine de convoi M.

La machine de convoi M est destinée à transporter des objets le long d'un parcours de convoi. Une telle machine de convoi M peut être utilisée sur une ligne de production dans de nombreux domaines (automobile, pharmaceutique, agroalimentaire, cartonnerie, etc.) ou encore au sein d'un système de tri d'objets tels que des colis, des lettres ou des déchets. Le parcours de convoi peut parcourir différents modules destinés à réaliser différentes actions respectives sur l'objet (par exemple peser un colis, lire un code-barre porté par le colis, etc.). Il peut comprendre des portions droites, des virages, des pentes, etc. Le parcours peut également passer à proximité de dispositifs particuliers, notamment des moteurs.

Par « objet instrumenté », on entend désigner un objet apte à être transporté par la machine de convoi M et doté d'un instrument ou système de mesure. L'objet est par exemple un colis, dans le cas d'une machine de convoi M pour un système de tri de colis, ou une structure de véhicule automobile dans le cas d'une machine de convoi M pour une ligne de production automobile.

L'instrument ou système de mesure est destiné à mesurer une ou plusieurs grandeurs physiques. Il comprend un ou plusieurs capteurs de mesure. La ou les grandeurs mesurées peuvent comprendre, de façon non exhaustive : une accélération, une distance, une inclinaison, un champ magnétique, une position angulaire, une température, un son, une mesure d'intensité lumineuse (diodes) et une pression. L'accélération peut être une accélération 3D. Elle est mesurée par un accéléromètre tri-axes. La distance peut, par exemple, être une distance par rapport à une paroi interne d'un module ou par rapport à une surface inférieure (fond d'un module ou sol). Elle peut être mesurée par un capteur de distance doté d'un émetteur et d'un récepteur de signaux lumineux, adapté pour mesurer le temps d'aller-retour d'un signal lumineux émis par l'émetteur, se réfléchissant sur la paroi interne du module et reçu par le récepteur. L'inclinaison peut être un angle d'inclinaison par rapport à l'horizontale. Elle peut être mesurée par un inclinomètre. Le champ magnétique peut être mesuré par un magnétomètre. La position angulaire peut être mesurée par un gyroscope. La température, le son et la pression peuvent être mesurés par un capteur thermique, un capteur acoustique et un capteur de pression, respectivement.

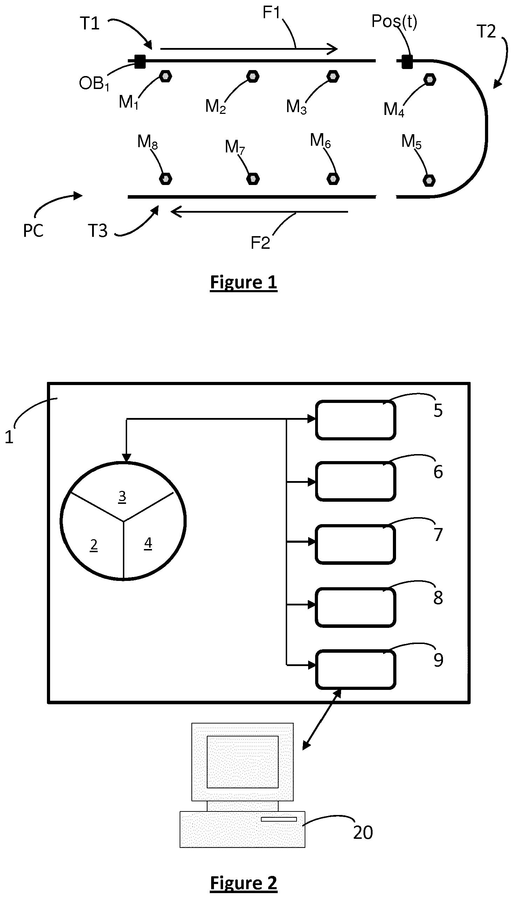

Sur la figure 2, on a représenté un schéma bloc fonctionnel d'un système de mesure 1, selon un exemple de réalisation particulier. Le système de mesure 1 comprend plusieurs capteurs 2-4, une batterie d'alimentation 5, une mémoire d'enregistrement 6, un microcontrôleur 7, une interface utilisateur 8 et une interface 9 de communication avec un dispositif d'analyse extérieur 20.

Dans l'exemple particulier décrit ici, les capteurs comprennent un composant 2 combinant un accéléromètre 3-axes et un magnétomètre 3-axes, un capteur de distance optique 3 et un capteur gyromètre 4. Le capteur de distance 3 comprend un élément émetteur d'un signal optique, un élément récepteur du signal optique émis, après réflexion de celui-ci sur une surface, un élément de calcul du temps aller/retour du signal (c'est-à-dire de la durée entre l'instant d'émission et l'instant de réception du signal, celui-ci ayant été réfléchi par une surface) et un élément de détermination de la distance entre le capteur 3 et la surface de réflexion.

L'interface de communication 9 comprend ici un connecteur mini-USB destiné à être connecté physiquement au dispositif d'analyse extérieur 20. En variante, elle pourrait comprendre une interface de communication radio destinée à communiquer par voie radio avec une interface de communication radio correspondante du dispositif d'analyse 20.

L'interface utilisateur 8 comprend ici :
- un bouton ON/OFF pour mettre en marche ou arrêter le système de mesure 1,
- un bouton START/STOP pour déclencher ou arrêter une acquisition de données de mesure et
- deux LEDs, l'une verte, destinée à signaler un bon fonctionnement, et l'autre rouge destinée à signaler soit une anomalie (signal rouge continu), soit un déchargement de la batterie (signal rouge clignotant).

Les capteurs 2-4, la batterie 5, la mémoire 6 et les interfaces 8 et 9 sont reliés au microcontrôleur 7 qui est destiné à contrôler le fonctionnement de ces éléments. Le microcontrôleur 7 est également relié à un bouton de « reset » (c'est-à-dire de réinitialisation) du microcontrôleur 7.

Tous les éléments du système de mesure, à l'exception du capteur de distance 3, sont montés sur une carte principale de circuit imprimé. Les éléments du capteur de distance 3 sont connectés à une carte secondaire de circuit imprimé, de plus petites dimensions que la carte principale. La carte secondaire est logée dans une encoche ménagée dans un bord (avant ou arrière dans le sens de déplacement du système de mesure) de la carte principale. Elle s'étend dans un plan perpendiculaire à celui de la carte principale.

La batterie peut être une pile bouton montée sur la carte principale. De façon alternative, elle peut être reliée à des connecteurs, prévus sur la carte principale, par un câble.

Le système ou instrument de mesure 1 présentent des caractéristiques techniques, notamment des dimensions (hauteur, largeur, épaisseur), un poids, une autonomie et une capacité mémoire, qui sont adaptés à l'application envisagée. L'autonomie doit notamment être suffisante pour permettre une prise de mesure durant la totalité du parcours de convoi.

Le dispositif d'analyse 20 comprend :
- une interface 200 de communication avec le système de mesure 1 ;
- une mémoire 201 contenant une table de correspondance T ;
- une mémoire 202 contenant des données relatives à une représentation spatiale prédéterminée relative à la machine de convoi M ;
- une mémoire 203 de stockage de données relatives à l'évolution temporelle d'une ou plusieurs grandeurs physiques mesurées par le système de mesure 1 porté un objet transporté par la machine de convoi M puis transmises au dispositif 20 ;
- un module 204 de reconnaissance d'empreintes correspondant à des caractéristiques de parcours de convoi ;
- un module 205 de détermination d'une chronologie de caractéristiques de parcours correspondant à des empreintes reconnues ;
- un module de localisation 206 ;
- un module de contrôle de fonctionnement 207 destiné à contrôler le bon fonctionnement de la machine de convoi M ;
- un module 208 de détection d'anomalie ;
- une interface utilisateur 209.

Tous les éléments 200-209 du dispositif d'analyse sont reliés à une unité centrale de commande 210, en l'espèce un microprocesseur, destiné à contrôler le fonctionnement de ces éléments. Les rôles de ces différents éléments apparaîtront dans la description du procédé qui suit. Le dispositif d'analyse 20 est dit « extérieur » du fait qu'il est situé en-dehors du ou des parcours de convoi de la machine de convoi M. Il s'agit ici d'un dispositif de type ordinateur distinct de la machine de convoi M. Le dispositif 20 comprend également un module de configuration, non représenté, destiné à configurer la dispositif d'analyse 20, notamment à enregistrer la table T et la représentation spatiale de la machine de convoi M dans les mémoires 201 et 202.

Le module de configuration (non représenté) et les modules 204 à 208 sont des modules logiciels comportant des instructions logicielles destinées à commander l'exécution des étapes de procédé correspondantes, explicitées plus loin, lorsque le module logiciel est exécuté par l'unité centrale de commande 210.

La table de données T, enregistrée dans la mémoire 201, contient, pour chaque caractéristique d'un ensemble de caractéristiques prédéfinies de parcours, des informations ou données de caractérisation d'une empreinte (ou signature) correspondant à la caractéristique de parcours considérée. Une caractéristique de parcours, tel qu'un virage ou un moteur situé à proximité, laisse une « empreinte », ou « signature », qui lui est propre, dans l'évolution temporelle d'une ou plusieurs grandeurs physiques appropriées. Par exemple, un moteur placé à proximité du parcours induit un pic d'intensité dans l'évolution temporelle de l'intensité du champ magnétique mesurée lors du passage à proximité du moteur par l'objet instrumenté. Des exemples de caractéristiques de parcours et d'empreintes correspondantes sont données ci-après :

**Table T**

| **Caractéristique de parcours** | **Empreinte** |
|---|---|
| Moteur à proximité | Pic d'intensité de champ magnétique |
| Virage | Plateau d'accélération |
| Changement de module | Pic de distance (correspond à un espace entre deux modules qui ne sont pas parfaitement adjacents) |
| Début de convoi | Transition entre un plateau de distance puis une phase de distance non stationnaire |
| Fin de convoi | Transition entre une phase de distance non stationnaire puis un plateau de distance |

L'empreinte peut être connue précisément a priori à partir d'informations relatives à la conception de la machine de convoi (par exemple l'empreinte d'une pente dans le parcours, la pente ayant une certaine inclinaison fixe). En variante, l'empreinte peut être déterminée lors d'un convoi préliminaire.

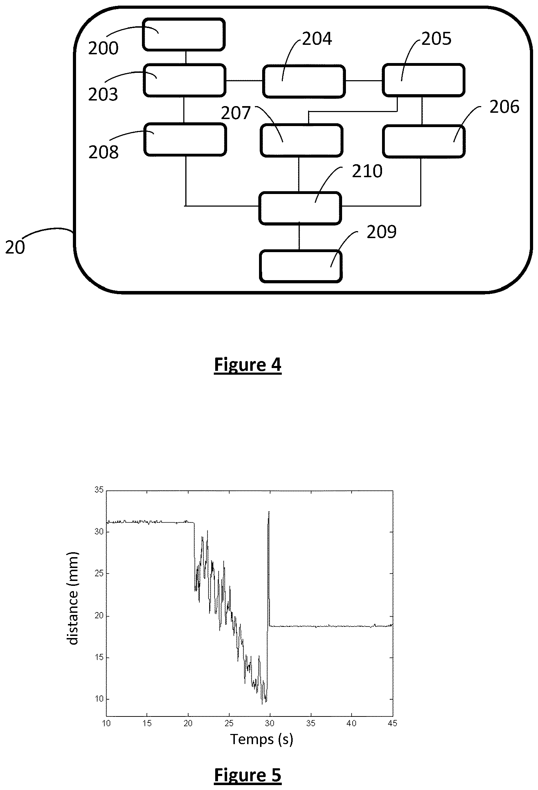

Sur les figures 5, 6A-6B et 7, on a représenté les évolutions temporelles de différentes grandeurs physiques (distance, accélération tri-axe et intensité de moteur électrique), mesurées par le système de mesure de l'objet OB₁ transporté par une machine de convoi, à titre d'exemples purement illustratifs seulement destinés à mieux faire comprendre la table de données T.

Sur la figure 5, on a représenté l'évolution temporelle d'une distance mesurée par un capteur de distance le long d'un parcours de convoi. La courbe représentée comprend trois phases : une première phase stationnaire (ou plateau) entre 10s et 20s, une deuxième phase intermédiaire non stationnaire, qui décroît globalement, entre 20s et 30s et une troisième phase stationnaire (ou plateau) au-delà de 30s. La première transition à 20s entre le premier plateau et la phase intermédiaire non stationnaire constitue l'empreinte de la caractéristique de parcours « début de convoi ». La deuxième transition à 30s entre la phase intermédiaire non stationnaire et le deuxième plateau constitue l'empreinte de la caractéristique de parcours « fin de convoi ».

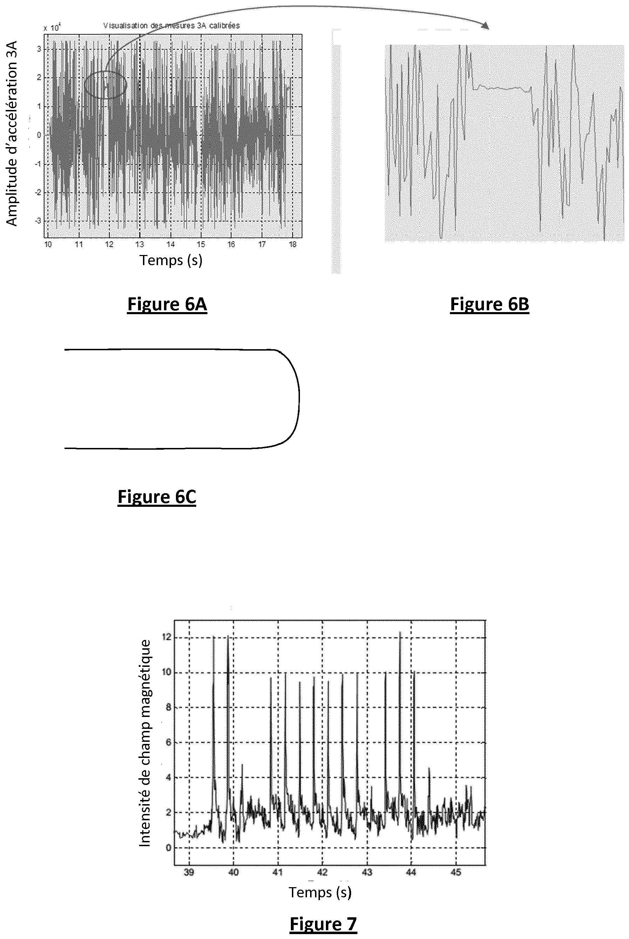

Sur la figure 6A, on a représenté l'évolution temporelle d'une accélération tri-axes mesurée par un capteur le long d'un parcours de convoi tel que représenté partiellement sur la figure 6C. Ce parcours comporte un virage à 180°. La figure 6B représente un zoom de cette courbe d'accélération entre les instants 11,4s et 12s. Sur le zoom de la figure 6C, on peut observer une phase stationnaire, c'est-à-dire un plateau, d'accélération autour de l'instant 11,7s. Ce plateau d'accélération constitue l'empreinte d'un virage dans le parcours de convoi.

Sur la figure 7, on a représenté l'évolution temporelle d'une intensité de champ magnétique mesurée par un capteur le long d'un parcours de convoi passant à proximité de douze moteurs. On peut observer sur cette courbe douze pics de champ magnétique d'intensité supérieure à 6 Gauss. Ces pics de champ magnétique constituent les empreintes de moteurs situés à proximité du parcours de convoi.

La table T est créée et enregistrée dans la mémoire 201 du dispositif d'analyse 20 lors d'une étape E0 de configuration du dispositif d'analyse. Cette configuration E0 peut être exécutée lors d'un couplage du dispositif d'analyse 20 avec la machine de convoi M.

La mémoire 202 contient des données relatives à une représentation spatiale prédéterminée relative à la machine de convoi M. Cette représentation spatiale comprend des données spatiales relatives à la machine, notamment au parcours de convoi ou à la pluralité de parcours de convoi qu'elle contient. Ces données spatiales peuvent être représentées sous la forme d'une carte ou d'un plan de la machine de convoi M. Cette carte ou ce plan fait apparaître les caractéristiques du ou des parcours de convoi : parties droites, virages, moteurs, etc. Les données relatives à la représentation spatiale de la machine de convoi M sont enregistrées dans la mémoire 202 lors de la configuration E0 du dispositif d'analyse et du couplage de celui-ci à la machine de convoi M.

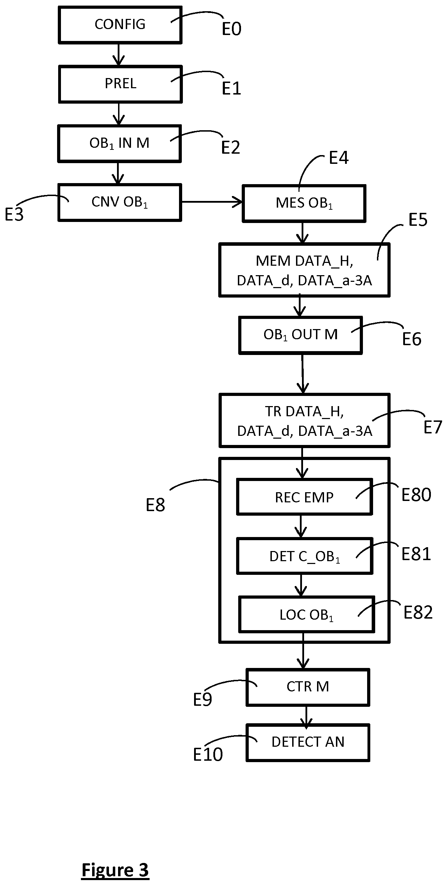

Un mode de réalisation particulier du procédé de localisation d'un objet instrumenté transporté le long d'un parcours par une machine de convoi M va maintenant être décrit en référence à la figure 3.

Lors d'une étape préliminaire E1, le système ou instrument de mesure 1 est placé sur un objet témoin OB₀ et fixé sur celui-ci par un scotch double face ou par tout autre moyen de fixation adapté. L'objet témoin OB₀ correspond à un objet apte à être convoyé par la machine de convoi M. Il s'agit par exemple d'un colis, dans le cas d'une application au tri de colis, d'une lettre, dans le cas d'une application au tri postal, d'un déchet, ou d'une structure de véhicule automobile dans le cas d'une application à une chaîne de production ou d'assemblage automobile.

L'objet témoin ainsi instrumenté, noté OB₁, est ensuite inséré dans la machine de convoi M, à l'entrée de celle-ci, lors d'une étape E2.

Le procédé comprend ensuite une étape de convoi E3, lors de laquelle l'objet témoin instrumenté est transporté par la machine de convoi M le long d'un parcours de convoi PC.

Le parcours de convoi PC comprend plusieurs tronçons successifs correspondant par exemple à différents modules destinés à réaliser différentes actions relatives à l'objet. Chaque portion présente des caractéristiques spécifiques. Par exemple, une portion peut :
- être linéaire ou comporter un ou plusieurs virage(s) ;
- être inclinée ou horizontale ;
- comporter un ou plusieurs moteurs.

Sur la figure 1, on a représenté, à titre d'exemple illustratif, une portion d'un parcours de convoi comportant trois tronçons T1, T2 et T3. Chaque tronçon du parcours de convoi correspond à un module de parcours. Les trois tronçons T1, T2 et T3 sont horizontaux. Les flèches F1 et F2 représentent le sens de déplacement d'un objet le long du parcours. Le premier tronçon T1 est linéaire, horizontal et passe à proximité de trois moteurs successifs M₁, M₂, M₃. Le deuxième tronçon T2 comporte deux virages et passe à proximité de deux moteurs M₄, situé juste avant le premier virage, M₅, situé juste après le deuxième virage, dans le sens de déplacement de l'objet. Le troisième tronçon T3 est linéaire et passe à proximité de trois moteurs successifs M₆, M₇, M₈.

Parallèlement à l'étape de convoi E3, le procédé comprend une étape E4 de mesure de l'évolution temporelle d'au moins une grandeur physique durant le transport de l'objet le long du parcours de convoi PC. Cette étape de mesure E4 est exécutée par le système de mesure 1 porté par l'objet OB₁. Dans l'exemple particulier décrit ici, le système 1 mesure :
- un champ magnétique H à l'aide du capteur accéléro-magnétomètre 2,
- une distance d à l'aide du capteur de distance optique 3,
- une accélération tri-axe a-3A à l'aide du capteur accéléro-magnétomètre 2.

Les données mesurées, de champ magnétique (DATA_H), de distance (DATA_d) et d'accélération (DATA_a-3A), sont enregistrées dans la mémoire 6 du système de mesure 1, lors d'une étape E5.

A la fin du transport de l'objet OB₁ par la machine de convoi M, l'objet OB₁ est sorti de la machine de convoi M, lors d'une étape E6. Le système de mesure 1 est séparé de l'objet témoin OB₀. Puis, lors d'une étape E7, les données de mesure enregistrées (DATA_H, DATA_d, DATA_a-3A) sont transmises au dispositif d'analyse 20, ici après connexion physique du système 1 au dispositif d'analyse 20. Les données de mesure transmises par le système 1 sont reçues par l'intermédiaire de l'interface de communication 200 et enregistrées dans la mémoire 203 du dispositif d'analyse 20.

Le procédé passe ensuite à une opération E8 d'analyse des données de mesure enregistrées.

L'opération d'analyse comprend une étape E80 de reconnaissance ou d'identification d'empreintes dans l'évolution temporelle des différentes grandeurs physiques mesurées. Cette étape E80 est mise en oeuvre par le module 204 de reconnaissance d'empreintes. Comme précédemment explicité, une empreinte correspond à une caractéristique de parcours.

L'étape de reconnaissance d'empreintes E80 est réalisée à l'aide de la table de données T enregistrée dans la mémoire 201 du dispositif d'analyse 20. La table de données T pourrait, de façon alternative, être accessible par le dispositif 20 à travers un réseau tel que l'Internet.

Lors de l'étape de reconnaissance E80, le dispositif d'analyse 20 peut reconnaître l'une au moins des empreintes du groupe comportant :
- un pic d'intensité de champ magnétique correspondant à un moteur électrique,
- un pic de distance correspondant à une transition d'un module à un autre module de la machine de convoi M et
- un plateau d'accélération correspondant à un virage.

Le procédé comprend ensuite une étape E81 de détermination d'une chronologie C_OB₁ de caractéristiques de parcours correspondant à des empreintes reconnues successives. Cette chronologie C_OB₁ correspond en définitive aux caractéristiques de parcours telle que perçues par l'objet instrumenté OB₁. L'étape E81 est mise en oeuvre par le module 205 du dispositif d'analyse.

Prenons l'exemple de la portion de parcours de convoi de la figure 1. Lors de l'étape de reconnaissance E80, le dispositif d'analyse 20 reconnaît successivement :
- trois pics successifs d'intensité de champ magnétique ;
- un pic de distance ;
- un pic d'intensité de champ magnétique ;
- un plateau d'accélération ;
- un pic d'intensité de champ magnétique ;
- un pic de distance ;
- trois pics successifs d'intensité de champ magnétique.

Lors de l'étape E81, le dispositif d'analyse 20 détermine la chronologie suivante C_OB₁ de caractéristiques de parcours, à partir des empreintes reconnues successives :
- trois moteurs successifs à proximité du parcours ;
- un changement de module ;
- un moteur à proximité du parcours ;
- un virage ;
- un moteur à proximité du parcours ;
- un changement de module ;
- trois moteurs successifs à proximité du parcours.

Dans la chronologie C_OB₁ déterminée, chaque caractéristique de la succession de caractéristiques de parcours déterminées peut être associée à des données temporelles correspondant à l'instant auquel l'objet OB₁ a perçu ou détecté la caractéristique de parcours considérée ou à la fenêtre temporelle durant laquelle l'objet OB₁ a perçu ou détecté la caractéristique de parcours considérée. Toutefois, la chronologie C_OB₁ pourrait ne contenir qu'une succession (c'est-à-dire une suite ordonnée) de caractéristiques de parcours.

Le procédé comprend ensuite une étape E82 de localisation de l'objet instrumenté OB₁. Cette étape E82 est mise en oeuvre par le module de localisation 206 du dispositif d'analyse 20. Lors de cette étape de localisation E82, on établit une correspondance entre la chronologie C_OB₁ déterminée et la représentation spatiale prédéterminée RS relative à la machine de convoi M. Cette correspondance permet de déterminer la position de l'objet OB₁ dans la machine de convoi M et plus particulièrement le long de son parcours de convoi, à chaque instant du convoi de l'objet OB₁.

La machine de convoi M peut comprendre plusieurs parcours de convoi possibles. Dans ce cas, l'étape de localisation E82 permet de déterminer, parmi l'ensemble des parcours possibles, le parcours emprunté par l'objet OB₁.

Supposons par exemple que l'on souhaite localiser l'objet OB₁ dans la machine de convoi M, et plus précisément le long de la partie de parcours de convoi représentée sur la figure 1, à un instant t donné, sachant que, à cet instant t, l'objet OB₁ se trouve au début du tronçon T2 à la position Pos(t) telle que représentée sur la figure 1. A cet effet, on détermine la chronologie C_OB₁. Cette chronologie pourrait être déterminée seulement partiellement. On pourrait par exemple déterminer seulement la partie de la chronologie entre le début du convoi et l'instant t. La chronologie C_OB₁ comprend, entre un instant de début de convoi et l'instant t, les caractéristiques de parcours suivantes : trois moteurs successifs à proximité du parcours, suivi d'un changement de module. Lors de l'étape de localisation E82, on détermine une correspondance entre la chronologie C_OB₁ déterminée (ou la partie de chronologie déterminée) et la représentation spatiale prédéterminée relative à la machine de convoi M. On en déduit que, à l'instant t, l'objet OB₁ se trouve au début du tronçon T2 sur la représentation spatiale.

Le procédé peut ensuite comprendre une étape E9 de contrôle du bon fonctionnement de la machine de convoi M. Cette étape E9 est mise en oeuvre par le module de contrôle 207 du dispositif d'analyse. Le contrôle de fonctionnement de la machine de convoi M peut être réalisé à partir de la chronologie C_OB₁ de caractéristiques de parcours déterminée. Il peut consister à vérifier si l'objet OB₁ emprunte le bon parcours, parmi un ensemble des parcours possibles. Il peut aussi consister à vérifier si la vitesse de convoi de l'objet OB₁ le long de son parcours de convoi, ou d'une partie de ce parcours, est conforme à une vitesse souhaitée. Il peut encore consister à vérifier que l'objet OB₁ suit correctement le parcours de convoi, c'est-à-dire qu'il est conforme à des certaines conditions ou paramètres prédéfinis de convoi.

Le procédé peut également comprendre une étape E10 de détection d'une anomalie dans le parcours de convoi de l'objet OB₁. Cette étape de détection E10 est mise en oeuvre par le module de détection 208 du dispositif d'analyse. La détection d'anomalie peut être basée sur une analyse de l'évolution temporelle d'une grandeur physique particulière mesurée par le système de mesure 1 durant le transport de l'objet OB₁ le long du parcours de convoi. Par exemple, il peut s'agir de l'inclinaison, notamment l'inclinaison d'un vecteur arbitraire, figé et attaché à l'objet convoyé, par rapport au plan orthogonal à la direction du champ de gravité terrestre (ou plan horizontal). de l'objet, définie par exemple par rapport à un plan horizontal. Sur la figure 7, on a représenté l'évolution temporelle d'une inclinaison mesurée par l'objet OB₁ lors de son convoi, à titre d'exemple purement illustratif. On constate une rupture dans l'évolution temporelle de l'inclinaison autour de l'instant 38s. Cette rupture correspond par exemple à un choc de l'objet OB₁ avec une pièce mal positionnée. On pourrait également détecter l'arrêt d'un moteur lors de l'étape E9, par détection de l'absence du pic d'intensité de champ magnétique correspondant.

## Revendications

1. Procédé de localisation d'un objet instrumenté (OB₁) transporté le long d'un parcours (PC) par une machine de convoi (M), ledit objet (OB₁) étant doté d'un système de mesure (1) d'au moins une grandeur physique donnée, comprenant une étape de mesure (E4), par le système de mesure (1), de l'évolution temporelle de ladite grandeur physique durant le transport de l'objet (OB₁) le long du parcours de convoi (PC), une étape (E80) de reconnaissance d'une pluralité d'empreintes, chaque empreinte correspondant à une caractéristique de parcours dans l'évolution temporelle de la grandeur physique mesurée, une étape (E81) de détermination d'une chronologie de caractéristiques de parcours (C_OB₁) correspondant à une succession d'empreintes reconnues et une étape (E82) de localisation de l'objet (OB₁) lors de laquelle on détermine une correspondance entre la chronologie de caractéristiques de parcours déterminée et une représentation spatiale prédéterminée relative à la machine de convoi (M).

2. Procédé selon la revendication précédente, **caractérisé en ce qu'**on mesure l'évolution temporelle de l'une au moins des grandeurs physiques du groupe comportant une accélération, une distance, une inclinaison, un champ magnétique, une position angulaire, une température, un son et une pression.

3. Procédé selon la revendication 1 ou 2, **caractérisé en ce que** la succession d'empreintes reconnues comprend différents types d'empreintes, notamment des empreintes du groupe comportant une accélération, une distance, une inclinaison, un champ magnétique, une position angulaire, une température, un son et une pression.

4. Procédé selon l'une des revendications précédentes, **caractérisé en ce que**, lors de l'étape de reconnaissance (E80), on reconnaît l'une au moins des empreintes du groupe comportant un pic d'intensité de champ magnétique mesuré correspondant à un moteur électrique, un pic de distance mesurée correspondant à une transition d'un module à un autre module de la machine de convoi (M) et un plateau d'une accélération mesurée correspondant à un virage.

5. Procédé selon l'une des revendications précédentes, **caractérisé en ce qu'**on mesure (E4) les évolutions temporelles d'une pluralité de grandeurs physiques.

6. Procédé selon l'une des revendications précédentes, **caractérisé en ce que** l'étape de reconnaissance (E80) est réalisée à l'aide d'une table de données (T) contenant, pour chaque caractéristique d'un ensemble de caractéristiques prédéfinies de parcours, des données de caractérisation de l'empreinte correspondant à ladite caractéristique de parcours.

7. Procédé selon l'une des revendications précédentes, **caractérisé en ce que**, la machine de convoi (M) comportant une pluralité de parcours de convoi possibles, lors de l'étape de localisation (E82), on détermine le parcours emprunté par l'objet instrumenté, parmi la pluralité de parcours de convoi possibles, à partir de la chronologie de caractéristiques de parcours déterminée.

8. Procédé selon l'une des revendications précédentes, **caractérisé en ce qu'**il comprend une étape (E9) de contrôle du bon fonctionnement de la machine de convoi (M) à partir de la chronologie de caractéristiques de parcours déterminée (C_OB₁).

9. Procédé selon la revendication précédente, **caractérisé en ce qu'**il comprend une étape (E10) de détection d'une anomalie dans le parcours de convoi de l'objet, basée sur une analyse de l'évolution temporelle d'une grandeur physique mesurée particulière.

10. Procédé selon l'une des revendications précédentes, **caractérisé en ce qu'**il comprend une étape (E7) de transmission de données d'évolution de la grandeur physique mesurée vers un dispositif d'analyse extérieur (20) et les étapes (E80) de reconnaissance d'au moins une empreinte et de localisation (E82) de l'objet (OB₁) sont exécutées par le dispositif d'analyse extérieur (20).

11. Dispositif d'analyse destiné à localiser un objet instrumenté transporté le long d'un parcours (PC) par une machine de convoi (M), ledit objet instrumenté étant doté d'un système (1) de mesure d'au moins une grandeur physique donnée, agencé pour mesurer l'évolution temporelle de ladite grandeur physique durant le transport de l'objet le long du parcours de convoi (PC), ledit dispositif d'analyse comprenant une interface de communication avec le système de mesure destinée à recevoir des données mesurées relatives à l'évolution temporelle de ladite grandeur physique durant le transport de l'objet le long du parcours de convoi, un module de reconnaissance (204) adapté pour reconnaître une pluralité d'empreintes, chaque empreinte correspondant à une caractéristique de parcours dans l'évolution temporelle de la grandeur physique mesurée, un module (205) de détermination d'une chronologie de caractéristiques de parcours correspondant à une succession d'empreintes reconnues et un module (206) de localisation de l'objet adapté pour établir une correspondance entre la chronologie de caractéristiques de parcours déterminée et une représentation spatiale prédéterminée relative à la machine de convoi (M) stockée en mémoire.

12. Système de localisation d'un objet transporté le long d'un parcours (PC) par une machine de convoi (M), **caractérisé en ce qu'**il comprend un objet instrumenté (OB₁) doté d'un système (1) de mesure d'au moins une grandeur physique donnée, agencé pour mesurer l'évolution temporelle de ladite grandeur physique durant le transport de l'objet le long du parcours de convoi (PC), et un dispositif d'analyse extérieur (20) selon la revendication précédente.

13. Système selon la revendication 12, **caractérisé en ce que** le dispositif d'analyse extérieur (20) comprend une mémoire (201) de stockage d'une table de données (T) contenant, pour chaque caractéristique d'un ensemble de caractéristiques prédéfinies de parcours, des données de caractérisation d'une empreinte correspondant à ladite caractéristique de parcours.

14. Système selon l'une des revendications 12 et 13, **caractérisé en ce que** le dispositif d'analyse extérieur (20) comprend un module (207) de contrôle du bon fonctionnement de la machine de convoi (M) à partir de la chronologie de caractéristiques de parcours déterminée.

15. Système selon l'une des revendications 12 à 14, **caractérisé en ce qu'**il comprend un module (208) de détection d'une anomalie, agencé pour détecter une anomalie dans le parcours de convoi de l'objet (OB₁) par analyse de l'évolution temporelle d'une grandeur physique mesurée particulière.

## Patentansprüche

1. Verfahren zur Ortung eines instrumentierten Objekts (OB₁), das von einer Fördermaschine (M) entlang eines Pfades (PC) transportiert wird, wobei das Objekt (OB₁) mit einem System zur Messung (1) wenigstens einer gegebenen physikalischen Größe ausgestattet ist, umfassend: einen Schritt der Messung (E4), durch das Messsystem (1), des zeitlichen Verlaufs der physikalischen Größe während des Transports des Objekts (OB₁) entlang des Förderpfades (PC), einen Schritt (E80) der Erkennung mehrerer Abdrücke, wobei jeder Abdruck einem Pfadmerkmal im gemessenen zeitlichen Verlauf der physikalischen Größe entspricht, einen Schritt (E81) der Bestimmung einer zeitlichen Abfolge von Pfadmerkmalen (C_OB₁), die einer Aufeinanderfolge von erkannten Abdrücken entspricht, und einen Schritt (E82) der Ortung des Objekts (OB₁), in welchem eine Entsprechung zwischen der bestimmten zeitlichen Abfolge von Pfadmerkmalen und einer vorbestimmten räumlichen Darstellung, die sich auf die Fördermaschine (M) bezieht, bestimmt wird.

2. Verfahren nach dem vorhergehenden Anspruch, **dadurch gekennzeichnet, dass** der zeitliche Verlauf wenigstens einer der physikalischen Größen der Gruppe gemessen wird, die eine Beschleunigung, eine Entfernung, eine Neigung, ein Magnetfeld, eine Winkelposition, eine Temperatur, einen Ton und einen Druck umfasst.

3. Verfahren nach Anspruch 1 oder 2, **dadurch gekennzeichnet, dass** die Aufeinanderfolge von erkannten Abdrücken verschiedene Typen von Abdrücken umfasst, insbesondere Abdrücke der Gruppe, die eine Beschleunigung, eine Entfernung, eine Neigung, ein Magnetfeld, eine Winkelposition, eine Temperatur, einen Ton und einen Druck umfasst.

4. Verfahren nach einem der vorhergehenden Ansprüche, **dadurch gekennzeichnet, dass** im Schritt der Erkennung (E80) wenigstens einer der Abdrücke der Gruppe erkannt wird, die einen Spitzenwert der gemessenen magnetischen Feldstärke, der einem Elektromotor entspricht, einen Spitzenwert der gemessenen Entfernung, der einem Übergang von einem Modul zu einem anderen Modul der Fördermaschine (M) entspricht, und ein Plateau einer gemessenen Beschleunigung, das einer Kurve entspricht, umfasst.

5. Verfahren nach einem der vorhergehenden Ansprüche, **dadurch gekennzeichnet, dass** die zeitlichen Verläufe mehrerer physikalischer Größen gemessen werden (E4).

6. Verfahren nach einem der vorhergehenden Ansprüche, **dadurch gekennzeichnet, dass** der Schritt der Erkennung (E80) mithilfe einer Datentabelle (T) durchgeführt wird, die für jedes Merkmal einer Menge von vordefinierten Pfadmerkmalen Charakterisierungsdaten des Abdrucks enthält, der dem betreffenden Pfadmerkmal entspricht.

7. Verfahren nach einem der vorhergehenden Ansprüche, **dadurch gekennzeichnet, dass**, wenn die Fördermaschine (M) mehrere mögliche Förderpfade aufweist, im Schritt der Ortung (E82) der Pfad, der von dem instrumentierten Objekt benutzt wird, unter den mehreren möglichen Förderpfaden anhand der bestimmten zeitlichen Abfolge von Pfadmerkmalen bestimmt wird.

8. Verfahren nach einem der vorhergehenden Ansprüche, **dadurch gekennzeichnet, dass** es einen Schritt (E9) der Kontrolle der ordnungsgemäßen Funktionsweise Fördermaschine (M) anhand der bestimmten zeitlichen Abfolge von Pfadmerkmalen (C_OB₁) umfasst.

9. Verfahren nach dem vorhergehenden Anspruch, **dadurch gekennzeichnet, dass** es einen Schritt (E10) der Detektion einer Anomalie im Förderpfad des Objekts umfasst, der auf einer Analyse des zeitlichen Verlaufs einer speziellen gemessenen physikalischen Größe basiert.

10. Verfahren nach einem der vorhergehenden Ansprüche, **dadurch gekennzeichnet, dass** es einen Schritt (E7) der Übertragung von Daten des gemessenen Verlaufs der physikalischen Größe zu einer externen Analysevorrichtung (20) umfasst und die Schritte (E80) der Erkennung wenigstens eines Abdrucks und der Ortung (E82) des Objekts (OB₁) von der externen Analysevorrichtung (20) ausgeführt werden.

11. Analysevorrichtung, welche dazu bestimmt ist, ein instrumentiertes Objekt zu orten, das von einer Fördermaschine (M) entlang eines Pfades (PC) transportiert wird, wobei das Objekt mit einem System zur Messung (1) wenigstens einer gegebenen physikalischen Größe ausgestattet ist, das dafür ausgelegt ist, den zeitlichen Verlauf der physikalischen Größe während des Transports des Objekts entlang des Förderpfades (PC) zu messen, wobei die Analysevorrichtung umfasst: eine Schnittstelle zur Kommunikation mit dem Messsystem, die dazu bestimmt ist, Messdaten zu empfangen, die sich auf den zeitlichen Verlauf der physikalischen Größe während des Transports des Objekts entlang des Förderpfades beziehen, ein Erkennungsmodul (204), das geeignet ist, mehrere Abdrücke zu erkennen, wobei jeder Abdruck einem Pfadmerkmal im gemessenen zeitlichen Verlauf der physikalischen Größe entspricht, ein Modul (205) zur Bestimmung einer zeitlichen Abfolge von Pfadmerkmalen, die einer Aufeinanderfolge von erkannten Abdrücken entspricht, und ein Modul (206) zur Ortung des Objekts, das geeignet ist, eine Entsprechung zwischen der bestimmten zeitlichen Abfolge von Pfadmerkmalen und einer in einem Speicher gespeicherten vorbestimmten räumlichen Darstellung, die sich auf die Fördermaschine (M) bezieht, herzustellen.

12. System zur Ortung eines Objekts, das von einer Fördermaschine (M) entlang eines Pfades (PC) transportiert wird, **dadurch gekennzeichnet, dass** es ein instrumentiertes Objekt (OB₁), das mit einem System (1) zur Messung wenigstens einer gegebenen physikalischen Größe ausgestattet ist, das dafür ausgelegt ist, den zeitlichen Verlauf der physikalischen Größe während des Transports des Objekts entlang des Förderpfades (PC) zu messen, und eine externe Analysevorrichtung (20) nach dem vorhergehenden Anspruch umfasst.

13. System nach Anspruch 12, **dadurch gekennzeichnet, dass** die externe Analysevorrichtung (20) einen Speicher (201) zur Speicherung einer Datentabelle (T) umfasst, die für jedes Merkmal einer Menge von vordefinierten Pfadmerkmalen Charakterisierungsdaten des Abdrucks enthält, der dem betreffenden Pfadmerkmal entspricht.

14. System nach einem der Ansprüche 12 und 13, **dadurch gekennzeichnet, dass** die externe Analysevorrichtung (20) ein Modul (207) zur Kontrolle der ordnungsgemäßen Funktionsweise der Fördermaschine (M) anhand der bestimmten zeitlichen Abfolge von Pfadmerkmalen umfasst.

15. System nach einem der Ansprüche 12 bis 14, **dadurch gekennzeichnet, dass** es ein Modul (208) zur Detektion einer Anomalie umfasst, das dafür ausgelegt ist, eine Anomalie im Förderpfad des Objekts (OB₁) durch Analyse des zeitlichen Verlaufs einer speziellen gemessenen physikalischen Größe zu detektieren.

## Claims

1. A method for locating an instrumented object (OB₁) transported along a path (PC) by a conveyor machine (M), said object (OB₁) being provided with a measurement system (1) for measuring at least one given physical quantity, including a step of measuring (E4), by the measurement system (1), the time evolution of said physical quantity during the transportation of the object (OB₁) along the conveyance path (PC), a step (E80) of recognizing a plurality of indicia, each indicium corresponding to a path characteristic in the time evolution of the measured physical quantity, a step (E81) of determining a timeline of path characteristics (C_OB₁) corresponding to a sequence of recognized indicia and a step (E82) of locating the object (OB₁) during which a correspondence is determined between the determined timeline of path characteristics and a predetermined spatial representation relating to the conveyor machine (M).

2. The method as claimed in the preceding claim, **characterized in that** the time evolution is measured of at least one of the physical quantities of the group comprising an acceleration, a distance, an inclination, a magnetic field, an angular position, a temperature, a sound, and a pressure.

3. The method as claimed in claim 1 or 2, **characterized in that** the sequence of recognized indicia includes different types of indicia, notably indicia of the group comprising an acceleration, a distance, an inclination, a magnetic field, an angular position, a temperature, a sound, and a pressure.

4. The method as claimed in one of the preceding claims, **characterized in that**, during the step of recognizing (E80), at least one of the indicia is recognized of the group comprising a measured magnetic field strength peak corresponding to an electric motor, a measured distance peak corresponding to a transition from one module to another module of the conveyor machine (M) and a measured acceleration plateau corresponding to a turn.

5. The method as claimed in one of the preceding claims, **characterized in that** the time evolutions of a plurality of physical quantities are measured (E4).

6. The method as claimed in one of the preceding claims, **characterized in that** the step of recognizing (E80) is performed with the aid of a data table (T) containing, for each characteristic of a set of predefined path characteristics, characterization data of the indicium corresponding to said path characteristic.

7. The method as claimed in one of the preceding claims, **characterized in that**, the conveyor machine (M) comprising a plurality of possible conveyance paths, during the step of locating (E82), the path taken by the instrumented object is determined, from among the plurality of possible conveyance paths, from the determined timeline of path characteristics.

8. The method as claimed in one of the preceding claims, **characterized in that** it includes a step (E9) of monitoring the correct operation of the conveyor machine (M) from the determined timeline of path characteristics (C_OB₁).

9. The method as claimed in the preceding claim, **characterized in that** it includes a step (E10) of detecting a fault in the conveyance path of the object, based on an analysis of the time evolution of a particular measured physical quantity.

10. The method as claimed in one of the preceding claims, **characterized in that** it includes a step (E7) of transmitting data on the evolution of the measured physical quantity to an external analysis device (20) and the steps (E80) of recognizing at least one indicium and locating (E82) the object (OB₁) are executed by the external analysis device (20).

11. An analysis device intended to locate an instrumented object transported along a path (PC) by a conveyor machine (M), said instrumented object being provided with a measurement system (1) for measuring at least one given physical quantity, arranged for measuring the time evolution of said physical quantity during the transportation of the object along the conveyance path (PC), said analysis device including a communication interface for communicating with the measurement system intended to receive measured data relating to the time evolution of said physical quantity during the transportation of the object along the conveyance path, a recognition module (204) suitable for recognizing a plurality of indicia, each indicium corresponding to a path characteristic in the time evolution of the measured physical quantity, a module (205) for determining a timeline of path characteristics corresponding to a sequence of recognized indicia and a locating module (206) for locating the object suitable for establishing a correspondence between the determined timeline of path characteristics and a predetermined spatial representation relating to the conveyor machine (M) stored in memory.

12. A system for locating an object transported along a path (PC) by a conveyor machine (M), **characterized in that** it includes an instrumented object (OB₁) provided with a measurement system (1) for measuring at least one given physical quantity, arranged for measuring the time evolution of said physical quantity during the transportation of the object along the conveyance path (PC), and an external analysis device (20) according to the preceding claim.

13. The system as claimed in claim 12, **characterized in that** the external analysis device (20) includes a memory (201) for storing a data table (T) containing, for each characteristic of a set of predefined path characteristics, characterization data of an indicium corresponding to said path characteristic.

14. The system as claimed in one of claims 12 and 13, **characterized in that** the external analysis device (20) includes a monitoring module (207) for monitoring the correct operation of the conveyor machine (M) from the determined timeline of path characteristics.

15. The system as claimed in one of claims 12 through 14, **characterized in that** it includes a fault detecting module (208), arranged for detecting a fault in the conveyance path of the object (OB₁), via analysis of the time evolution of a particular measured physical quantity.
